# EUROPEAN PATENT APPLICATION

(11) **EP 4 014 969 A1**
(43) Date of publication of application: **22.06.2022**
(21) Application number: 20214654.4
(22) Date of filing: 16.12.2020
(51) Int. Cl.: A61K 9/19, A61K 47/24, A61K 47/26

(54) **AQUEOUS SOLUTION**

(71) Applicant: EVER Valinject GmbH, 4866 Unterach am Attersee (AT)
(72) Inventor: ACHLEITNER, Maria-Lena, 4866 Unterach am Attersee (AT); HESSENBERGER, Michael, 4866 Unterach am Attersee (AT); SCHNAIT, Heinz, 4866 Unterach am Attersee (AT)
(74) Representative: SONN Patentanwälte OG

(57) **Abstract**

The present invention relates to an aqueous solution comprising glycopeptide antibiotics, selected from the group consisting of vancomycin, telavancin, oritavancin, teicoplanin, dalbavancin and mixtures thereof, especially vancomycin, and N-acetylated D-cysteine (D-aCys), the solution having a pH of from about 4.0 to 7.0 and preferably being present in a suitable container with air as headspace. The invention relates further to the use of such an aqueous solution as a medicament, especially for use in the treatment of bacterial infections, and a method for stabilizing glycopeptide antibiotics, especially vancomycin.

## Description

Vancomycin is a tricyclic glycopeptide antibiotic and is used to treat a number of bacterial infections. It was first sold in 1954, is on the World Health Organization's List of Essential Medicines and classified as critically important for human medicine.

Vancomycin acts by inhibiting proper cell wall synthesis in Gram-positive bacteria. The large hydrophilic molecule of vancomycin is able to form hydrogen bond interactions with the terminal D-alanyl-D-alanine moieties of the NAM/NAG-peptides. Under normal circumstances, this is a five-point interaction. This binding of vancomycin to the D-Ala-D-Ala prevents cell wall synthesis of the long polymers of N-acetylmuramic acid (NAM) and N-acetylglucosamine (NAG) that form the backbone strands of the bacterial cell wall, and it prevents the backbone polymers that do manage to form from cross-linking with each other.

Vancomycin is monographed in the European Pharmacopoeia (Ph.Eur., EP) and in the United States Pharmacopoeia (USP). Vancomycin is obtained through fermentation, extraction and purification.

In pharmaceutical use, vancomycin is usually administered as the hydrochloride salt. This salt has previously been supplied for oral and parenteral use as a dry solid or as a frozen liquid preparation. Liquid, especially aqueous formulations of vancomycin hydrochloride would be desirable but have limited stability which limits their practical use.

Many attempts have since been made to stabilize vancomycin and related glycopeptide antibiotics in liquid preparations.

WO 97/19690 discloses stable solutions of vancomycin hydrochloride comprising between about 0.5 % and about 12 % w/v vancomycin hydrochloride and between about 0.5 % and about 30 % v/v ethanol. These solutions are said to be particularly useful for storage in a liquid state not requiring either freezing or freeze drying in order to maintain stability of the active agent.

WO 2014/194296 A1 relates to vancomycin containing compositions substantially free of precipitation after at least about 12 months of storage at refrigerated or ambient conditions, the compositions include vancomycin or a pharmaceutically acceptable salt thereof; a polar solvent including propylene glycol, polyethylene glycol and mixtures thereof; lactic acid, a lactate salt, or mixtures thereof; and optionally a pH adjuster in an amount sufficient to maintain a pH of the compositions at from about 3 to about 8.

WO 2001/82971 A discloses pharmaceutical compositions which contain a cyclodextrin such as sulfobutylether-betacyclodextrin and a glycopeptide antibiotic such as vancomycin or a salt thereof, the composition comprising 60 to 99 wt.-% of water.

JP 2008 8201778 A discloses aqueous vancomycin solutions with glycerol and alanine or lactic acid. It is further disclosed in this document that vancomycin degrades rapidly in aqueous solutions and therefore, freeze-drying is necessary to secure long-term stability.

WO 2014/085526 discloses stabilized lipid-based vancomycin compositions, wherein amino acids or derivatives thereof stabilize vancomycin.

US 8 778 873 discloses a stability study for a combination of ceftriazone and vancomycin at pH 8.8. L-arginine, L-lysine and L-histidine are disclosed as suitable stabilizing agents.

US 10 039 804 B2 relates to solutions comprising a glycopeptide antibiotic, for example vancomycin, and an amino acid or amino acid derivative such as N-acetyl-glycine or N-acetyl-D-alanine. These solutions are said to be stable or stabilized for long-term periods at conditions of normal use and storage, and can be formulated as pharmaceutical solutions for use in subjects. Methods of manufacturing and using these solutions are also provided, as are methods of stabilizing a glycopeptide antibiotic, for example vancomycin, using amino acids or amino acid derivatives such as N-acetyl-glycine or N-acetyl-D-alanine.

WO 2017/194385 A1 discloses liquid formulations of glycopeptide antibiotics or pharmaceutically acceptable salts thereof, wherein the glycopeptide antibiotic is selected from vancomycin, telavancin, oritavancin, teicoplanin and dalbavancin. Said formulations are said to be suitable as infusion solutions or as a concentrate for making infusion solutions and are stabilized with sulfobutyl ether betacyclodextrin. An organic solvent (e.g. ethanol, propylene glycol, etc.) and, in addition, N-acetyl-D-alanine, N-acetyl-glycine and amino acids can be included in the formulation.

There still remains a need for solutions of vancomycin and related glycopeptides, like telavancin, oritavancin, teicoplanin and dalbavancin, which solution is an aqueous solution and possesses an improved long-term stability under conditions of normal use and storage, and which remain suitable for administration to a subject throughout their stability period.

It has now surprisingly been found that aqueous solutions comprising glycopeptide antibiotics, like vancomycin, telavancin, oritavancin, teicoplanin, dalbavancin, and mixtures thereof, especially vancomycin, can successfully be stabilised by addition of N-acetylated D-cysteine. Under comparable conditions, aqueous solutions comprising similar amounts of vancomycin and stabilizing agents showed in three-month trials significantly lesser amounts of the two major degradation products of vancomycin (see WO 2017/194385 A1) known as VANC-[61-Carboxy]-antichloro (also known as "CDP1 minor"; "CPD-I-m" or "Impurity E" (Ph. Eur.)) and VANC-[61-Carboxy]-synchloro (also known as "CDP1 major"; "CPD-I-M" or "Impurity B" (Ph. Eur.)) when stabilised with N-acetylated D-cysteine (D-aCys), compared to a stabilisation with N-acetylated D-alanine (D-aAla).

Thus, the present invention is directed to an aqueous solution comprising glycopeptide antibiotics, selected from the group consisting of vancomycin, telavancin, oritavancin, teicoplanin, dalbavancin and mixtures thereof, especially vancomycin, and N-acetylated D-cysteine, the solution having a pH of from about 4.0 to about 7.0. Preferably, the solution is present in a suitable container for parenteral administration, such as vials, syringes or polymer infusion bags and "ready-to-use" IV containers.

The pH of the glycopeptide antibiotic solution according to the present invention (measured at approximately 25°C) can be adjusted in any suitable manner by means of the addition of pH adjusting agents known in the prior art in an amount sufficient to maintain a pH of the solution at the desired range from about 4.0 to about 7.0, especially between about 5.0 and about 6.5, more preferred between about 5.3 and about 6.2, e.g. by addition of aqueous hydrochloric acid solutions or aqueous sodium hydroxide solutions. Such solutions can be diluted or concentrated. When comparing stabilisation with N-acetylated D-cysteine (D-aCys) to a stabilisation with N-acetylated D-alanine (D-aAla), use of similar amounts resulted surprisingly in virtually the same stability over a three-month test period.

The term "aqueous solutions" as used in the context of the present invention refers to any solution in which water is either the only solvent or one of the main solvents (equal or above 50% V/V). Aqueous solutions include, but are not limited to solutions comprising 50%, 60%, 70%, 80%, 85%, 90%, 95%, 98%, 99% V/V water. The aqueous solutions can additionally comprise pharmaceutically acceptable and physiologically tolerated organic solvents, either one or more. Such organic solvents include a glycol - such as polyethylene glycol (PEG 200, PEG 300, PEG 400, PEG 600, PEG 4000 etc.) and propylene glycol, and alcohols such as ethanol, or any mixtures thereof. The aqueous solution(s) according to the present invention may further comprise diluents selected from a group consisting of sterile water, bacteriostatic water for injection (BWFI), a pH buffered solution (e.g. phosphate-buffered saline), sterile saline solution, Ringer's solution, dextrose solution, Normosol^{®}-M and ISOLYTE^{®} E, and any mixtures thereof.

According to a preferred embodiment of the present invention, the aqueous solution has a pH of between about 4.0 to about 7.0, especially between about 5.0 and about 6.5, more preferred between about 5.3 and about 6.2, and is present in a suitable container with air as headspace. Most surprisingly, with the pH of the solution at between 4.0 and 6.0 and 21% headspace oxygen (that is, no protective gas blanket or any other sort of added inert gas in the test vial), use of N-acetylated D-cysteine (D-aCys) as stabilizing agent, resulted in the stability of the solution being even better than compared to use of acetylated D-alanine (D-aAla) as known from the state-of-the-art comparative product at pH 5.0 and 0% or 10.5% headspace oxygen, with only a slightly increased concentration of N-acetylated D-cysteine (D-aCys) over D-aAla.

The present invention also provides for the above-mentioned aqueous solution(s) for use as a medicament, especially for use in the treatment of bacterial infections.

As already mentioned, the aqueous solution according to the present invention is suitable for storage in liquid state, thus it can be stored in vials, syringes and "ready to use" IV containers, as are known in the art. Liquid aqueous solutions of glycopeptide antibiotics according to the present invention can also be provided as stable ready-to-use concentrate formulations, which concentrates may be diluted prior to administration to the patient.

When administering the aqueous solution according to the present invention to patients, there is no need for the step of reconstituting a lyophilized API (e.g. vancomycin) powder; however, the content of vials and syringes could be further diluted to target concentration prior to administration. Suitable diluents for solutions of the invention include any known diluent acceptable for pharmaceutical use (e.g., intravenous administration); for example, water, physiological saline, 5% dextrose solution, lactated Ringer's solution or combinations thereof.

The present invention further provides a method for stabilizing glycopeptide antibiotics, selected from the group consisting of vancomycin, telavancin, oritavancin, teicoplanin, dalbavancin and mixtures thereof, especially vancomycin, which involves addition of N-acetylated D-cysteine (D-aCys) to a solution comprising the glycopeptide antibiotic(s), especially vancomycin, or addition of such glycopeptide antibiotic(s), especially vancomycin, to a solution comprising N-acetylated D-cysteine (D-aCys).

Generally, N-acetylated D-cysteine (D-aCys) will be present in the aqueous solution according to the invention in a 20 to 35 fold molar surplus over the active pharmaceutical ingredient of the glycopeptide antibiotic, especially vancomycin.

The present invention will now be further explained by the following examples, to which it should not be limited. The term API as used in the following examples refers to the active pharmaceutical ingredients used to represent glycopeptide antibiotics, namely vancomycin. The disclosure of the examples is also applicable to other glycopeptide antibiotics, like telavancin, oritavancin, teicoplanin, dalbavancin and mixtures thereof.

**Abbreviations**

| | | |
|---|---|---|
| - | aAla | N-Acetyl-D-alanine |
| - | a-L-Ala | N-Acetyl-L-alanine |
| - | aAla_Raz | Racemate; mixture of N-Acetyl-D-alanine and N-Acetyl-L-alanine |
| - | aCys | N-Acetyl-D-cysteine |
| - | a-L-Cys | N-Acetyl-L-cysteine |
| - | aCys_Raz | Racemate; mixture of N-Acetyl-D-cysteine and N-Acetyl-L-cysteine |
| - | DoE | Design of Experiment |
| - | HSO | Oxygen in Headspace |
| - | Ref | Reference (formulation without any amino acid) |

The drug product is usually available as lyophilized vancomycin powder for solution which requires reconstitution with sterile water before use. After reconstitution, each vial contains 50 mg/mL of vancomycin hydrochloride. Before intravenous (i.v.) administration, the reconstituted solution is then further diluted with sodium chloride 0.9 % (w/w) or glucose 5 % (w/w) solution for injection.

In order to minimize the effort for the health care professionals and a potential contamination risk, the aqueous solution according to the present invention is preferably present as a ready-to-use formulation with a concentration of 5 mg/mL. As mentioned above, higher concentrations are of course also possible, which should then be diluted prior to application to the desired concentration range. In the following examples, the aqueous solutions each have a concentration of 5 mg vancomycin per mL solution.

Excipients like lactic acid or acetylated glycine enhances the stability of Vancomycin in water. These excipients are known to interact with specific functional groups of Vancomycin, as disclosed in Loll, Patrick J.; et al.; Vancomycin Binding to Low-Affinity Ligands: Delineating a Minimum Set of Interactions Necessary for High-Affinity Binding; J. Med. Chem.; 1999.

In the following examples a commercially available API was used. API dissolved in water (without stability enhancing excipients) acted as reference to assess the effect on the stability.

Also, a commercially available drug product contains acetylated D-alanine to stabilize Vancomycin (based on WO 2017/194385 A1). Additional reference solutions were compounded to demonstrate the effect of alternative excipients to stabilize glycopeptide antibiotics.

### Materials

The used materials are listed in Table 1.

**Table 1: Materials used**

| **Name** | **Batch (Lot) number** | **Supplier** |
|---|---|---|
| 1 N NaOH | HC988427 | Merck |
| 5 N NaOH | HC68506913 | Merck |
| 1 N HCl | HC734538 | Merck |
| clear glass vials; 6R 5mL | 81005518-107-01 | Müller+Müller |
| Coated rubber stopper; 20 mm; West Flurotec coated | 1182060287 | Westar |
| Crimping cap | 9182017791 | Altmann |
| Syringe filter 0.45 m PVDF PN4408T | 3630880A | PALL |
| Vancomycin hydrochloride "as-is": 92.71% | 97190102 | Chongqing Daxin Pharmaceuticals |
| N-acetyl-D-Alanine (aAla) | 5002R23N | Alfa Aesar |
| N-acetyl-D-cysteine (aCys) | AGN20-51984 | Angene |
| N-acetyl-L-cysteine | WXBC0011V | Sigma Aldrich |
| N-Acetyl-L-Alanine | BCCC1602 | Sigma Aldrich |

### Equipment

Table 2 shows the used equipment.

**Table 2: Equipment used**

| **Name** |
|---|
| Water purification system (Satorius Stedim) |
| Pipette (Multipette Xstream) |
| Analytical balance XP 205DR/M (Mettler Toledo) |
| pH-meter inoLAB 7310 P (WTW) |
| Magnetic stirrer |
| HPLC 1290 (Agilent) |
| Glovebox LAB master Pro ECO (M.Braun) |
| Oxygen Analyzer fms-760 (Lighthouse Instruments) |

### Methods

The following methods were applied:

| | |
|---|---|
| - pH-value: | acc. to internal working instruction (Ph.Eur. 2.2.3) |
| - Assay and chromatographic purity: | acc. to in-house method |
| - Oxygen in headspace: | acc. to internal working instruction |

Table 3 shows the HPLC method used for assay and chromatographic purity. The determination of assay and purity is based on the respective total peak area. No reference standard was used.

The internal reporting limit (LoR) was set to ≥ 0.05%.

**Table 3: HPLC-method for assay and purity**

| | | | | | | | | |
|---|---|---|---|---|---|---|---|---|
| The method is a local implementation of a published Ph.Eur. method | | | | | | | | |

| **Parameters** | **Settings** | | | | | | | |
|---|---|---|---|---|---|---|---|---|
| Column | Aquity CSH C18 2.1 x 150 mm; 1.7 µm (Waters) | | | | | | | |
| Mobile Phase | A: AcN : MeOH : solution A (3:4:93 v/v/v %) | | | | | | | |
| | B: AcN : MeOH : solution A (10:40:50 v/v/v %) | | | | | | | |
| Solution A | 7.0 g of tris (hydroxymethyl) aminomethane R is weighed into a 1 L beaker and dissolved in approximately 950 mL ultrapure water. The pH is adjusted to the target pH in the range 8.0-8.3, as described in Table 1058.-1 (acc. to EP monograph) using a 20 % (V/V) solution of glacial acetic acid R in water. The solution is finally diluted to 1000 mL with ultrapure water in a 1 L volumetric flask. | | | | | | | |
| Gradient | t [min] | 0 | 7 | 21 | 35 | 37 | 37.5 | 45 |
| | A [%] | 88 | 88 | 75 | 25 | 25 | 88 | 88 |
| | B [%] | 12 | 12 | 25 | 75 | 75 | 12 | 12 |
| Sample concentration | 5 mg/mL | | | | | | | |
| Flow | 0.3 mL/min | | | | | | | |
| Column temperature | 40 °C | | | | | | | |
| Sample Rack temperature | 5 °C | | | | | | | |
| Detector Wavelength | 280 nm | | | | | | | |
| Injection volume | 2 µL | | | | | | | |
| Run time | 45 min | | | | | | | |

Table 4 describes the relative retention times of measured impurities from the European Pharmacopoeia literature, also giving the internal names of these impurities.

A reference solution "Vancomycin for system suitability CRS" (as described in Ph. Eur. monograph) was used to assign the respective impurity.

**Table 4: Relative retention times (RRTs) of impurities and internal names of the impurities**

| **Relative retention times (RRTs) acc.to Ph. Eur.** | **Impurity name acc. to Ph. Eur.** | **Internal name of impurity** |
|---|---|---|
| 0.37 | Impurity E | VANC-[61-Carboxy]-antichloro |
| 0.70 | Impurity B | VANC-[61-Carboxy]-synchloro |

### Experimental Details

### Excipients

Tested in connection with vancomycin in the course of the present invention are the selected stabilising agents described in Table 5.

**Table 5: Overview of stabilising agents used for vancomycin.**

| **Description** | **Molecular weight** | **Chemical structure** |
|---|---|---|
| N-acetyl-D-alanine | 131.1 g/mol | 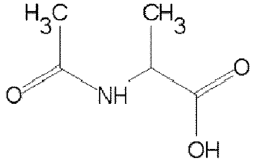 |
| C₅H₉NO₃ | | |
| CAS: 19436-52-3 | | |
| N-acetyl-L-alanine | | |
| C₅H₉NO₃ | | |
| CAS: 97-69-8 | | |
| N-acetyl-D-cysteine | 163.2 g/mol | 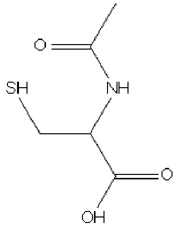 |
| C₅H₉NO₃S | | |
| CAS: 26117-28-2 | | |
| N-acetyl-L-cysteine | | |
| C₅H₉NO₃S | | |
| CAS: 616-91-1 | | |

### Composition of samples

An excess amount of the respective stabilising agent was used to guarantee that all API molecules have a chance to bind to the potential ligands. For compounding of the sample with acetylated alanine, a 20-fold excess of the stabilising agent was used in relation to the molar amount of vancomycin (which is 3.45 µM (or 5 mg/mL)) as API. Water and pH adjusters (NaOH / HCl) were added up to the required concentration and pH.

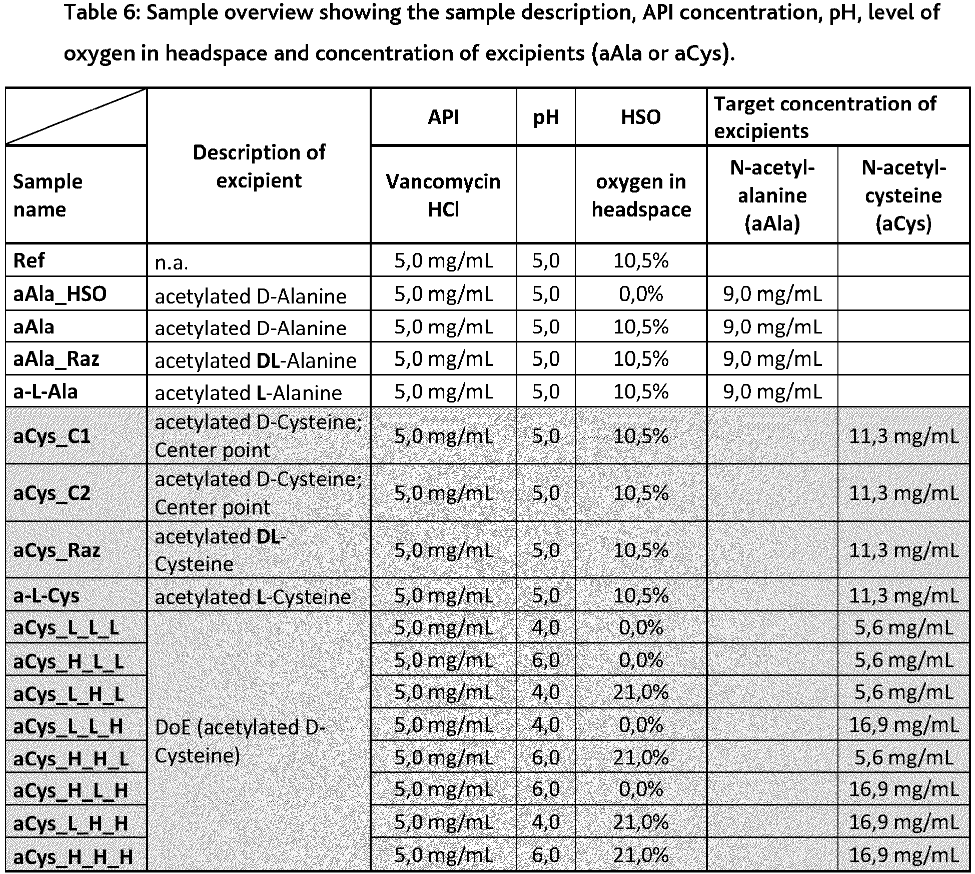

In order to evaluate the influence of HSO, pH and concentration of stabilising agent (D-aCys), a DoE setup was applied with the following target values.

The DoE (Design of Experiment) samples (see Table 6) are labelled according to the varied parameters pH, HSO and concentration of acetylated D-cysteine as "high" (H) or "low" (L). A full factorial design was chosen which allows for statistical evaluation of the effect of each parameter and also the forecast for the stability of sample with intermediate settings (within the chosen design space; see Table 7).

**Table 7: Parameter setting for DoE setup for sample with pAla**

| **Parameter** | **Low (-)** | **Center Point (C)** | **High (+)** |
|---|---|---|---|
| pH | 4.0 | 5.0 | 6.0 |
| HSO | 0% | 10.5 % | 21.0 % |
| Concentration of excipient | 10-fold molar to API: D-aCys: 5.6 mg/mL | 20-fold molar to API: D-aCys: 11.3 mg/mL | 30-fold molar to API: D-aCys: 16.9 mg/mL |

The whole possible range for oxygen in headspace was covered (0%, 10.5%, 21%). For pH, the reasonable range for a stable condition of the API was chosen. The concentration for the respective excipient was based on the molar ratio to the API.

The center point of each DoE was compounded twice to assess the variability of results.

The samples containing no excipient, "aAla" or a racemic mixture of D- and L-forms ("Raz") of stabilising agents are used as reference in order to assess the effect of D-aCys in comparison. The sample "aAla_HSO" was prepared to have results for this excipient at lower oxygen level (i.e. 10.5 %) in headspace.

For these reference samples, the center point settings for the DoE setup are used (see Table 7).

As the racemate of aAla was commercially not available, a 1:1 mixture of the respective D- and L-form was used.

A final concentration of 5 mg/mL of vancomycin was targeted (considering the "as is" content of the respective API batch according to the provided CoA).

### Compounding

The matrix solution (purified water + excipient(s)) was prepared according to the composition stated in Table 6. The API is added once all components are visually dissolved (clear solution).

pH was adjusted using 1 M or 5 M NaOH or 1 M HCl.

In parallel, matrix solutions (without API) of single excipients were prepared in the concentrations shown in Table 6 and analysed in parallel (at staring point) using HPLC. Peaks present in the chromatogram can be attributed to the respective excipient and were considered during data analysis of the respective sample.

### Filling

The compounded solutions are filtered through a PVDF syringe filter (0.45 µm pore size) and filled into clear glass vials which were closed with a coated rubber stopper under normal atmosphere.

The respective oxygen concentration in the headspace (HSO) of the vials was adjusted within a glovebox. Vials with a target of 21 % of oxygen are filled under atmospheric conditions (outside of the glovebox).

After filling, all samples were checked for visible particles in front of a black & white board before placing the samples under the respective storage condition.

Also, the oxygen concentration in the headspace of vials filled in the glovebox was confirmed (e.g. by using a Lighthouse oxygen analyzer).

### Stability conditions and schedule

Samples were stored at 25 °C and 40 °C. During previous studies, it turned out that storage at higher temperature did not result in a useful outcome (i.e. formation of particles and degradation of > 10 % of assay).

The stability testing was conducted at the following time points (see Table 8). Samples were stored upright and single samples were retrieved from the respective storage condition to conduct the scheduled analysis.

**Table 8: Time schedule for stability testing; "x" marks the intended testing time point for the respective storage condition**

| **Storage temperature** | **2 weeks** (completed) | **1 month** (completed) | **3 months** (completed) | **6 months** (open) | **12 months** (open) |
|---|---|---|---|---|---|
| 25 °C | x | x | x | x | x |
| 40 °C | x | | | | |

Vials (including one vial for analysis at starting point (T0)) with a minimum volume of 3 mL were filled per sample set.

### RESULTS

### pH-value

The results of pH measurements at T0 and after 2 weeks at 25°C and 40°C, 1 month at 25°C as well as after 3 months at 25°C are summarized in Table 99.

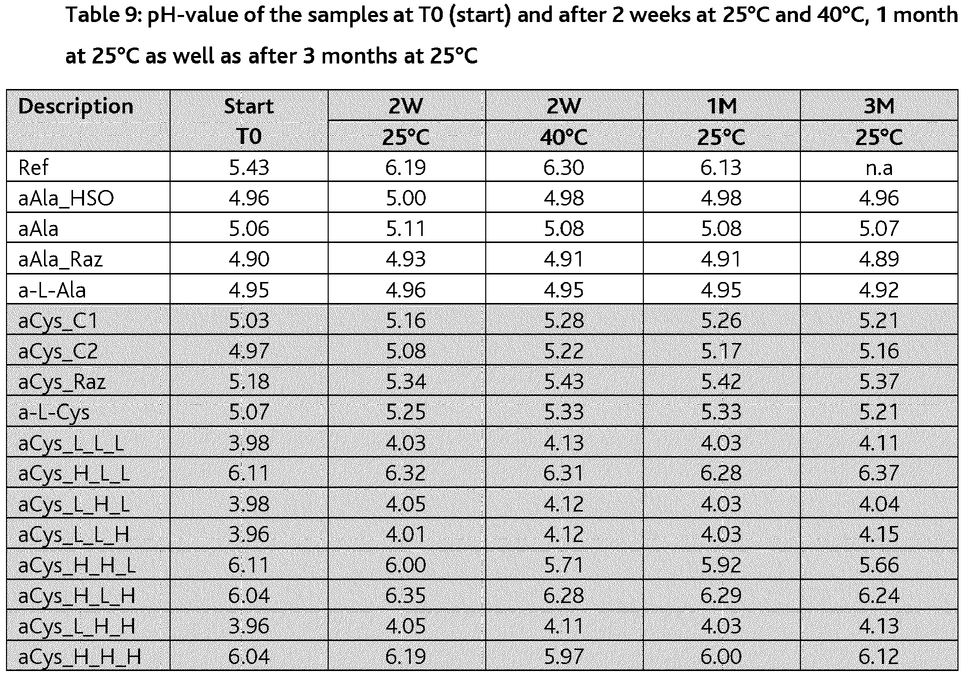

### Content and purity of Vancomycin

Table 100 shows the results of the content analysis of the samples at T0 and after 2 weeks at 25°C and 40°C, 1 month at 25 °C as well as after 3 months at 25°C. The peak area of the main peak was determined expressed as area % of total area. The start value was set to 100% and the following results were re-calculated accordingly.

Due to the observed precipitation (related to the degradation of the API), no analysis was conducted for sample "Ref" after 3 months at 25 °C.

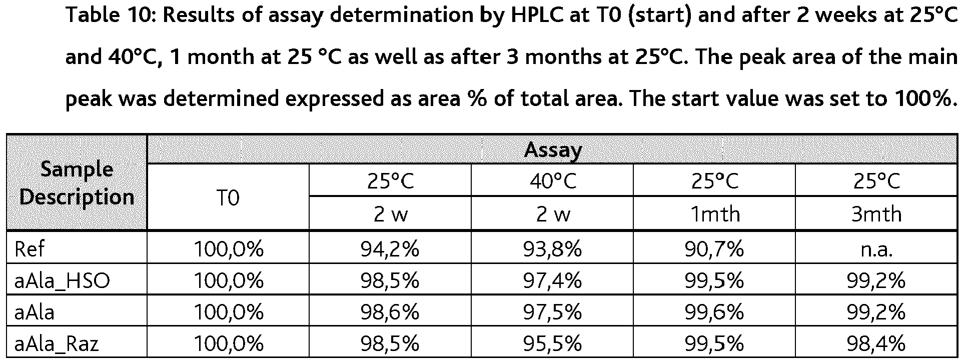

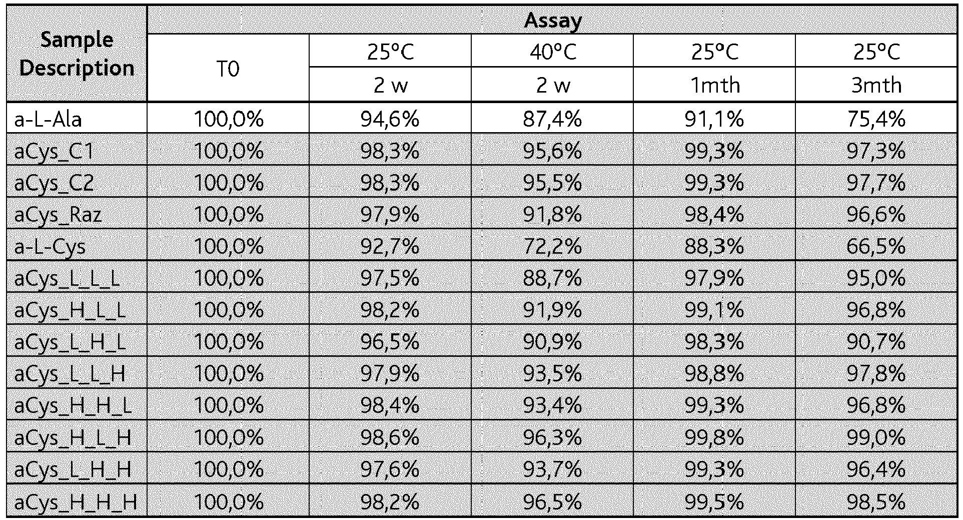

Table 11 shows the sum of the detected impurities (based on area %) per sample setting at T0 and after 2 weeks at 25°C and 40°C, 1 month at 25 °C as well as after 3 months at 25°C.

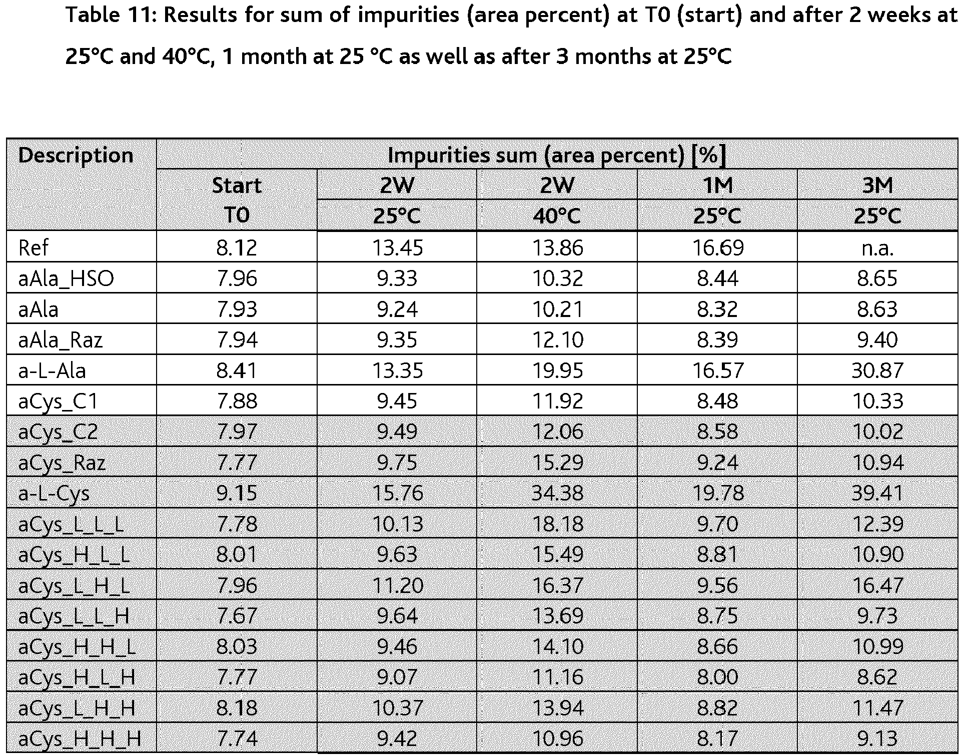

The evaluation of the stability of the drug product focuses on the change of main degradation products.

Table 12 provides an overview of the change of the main degradation products of vancomycin in comparison to T0 after 2 weeks at 25°C and 40°C, after 1 month as well as 3 months at 25 °C.

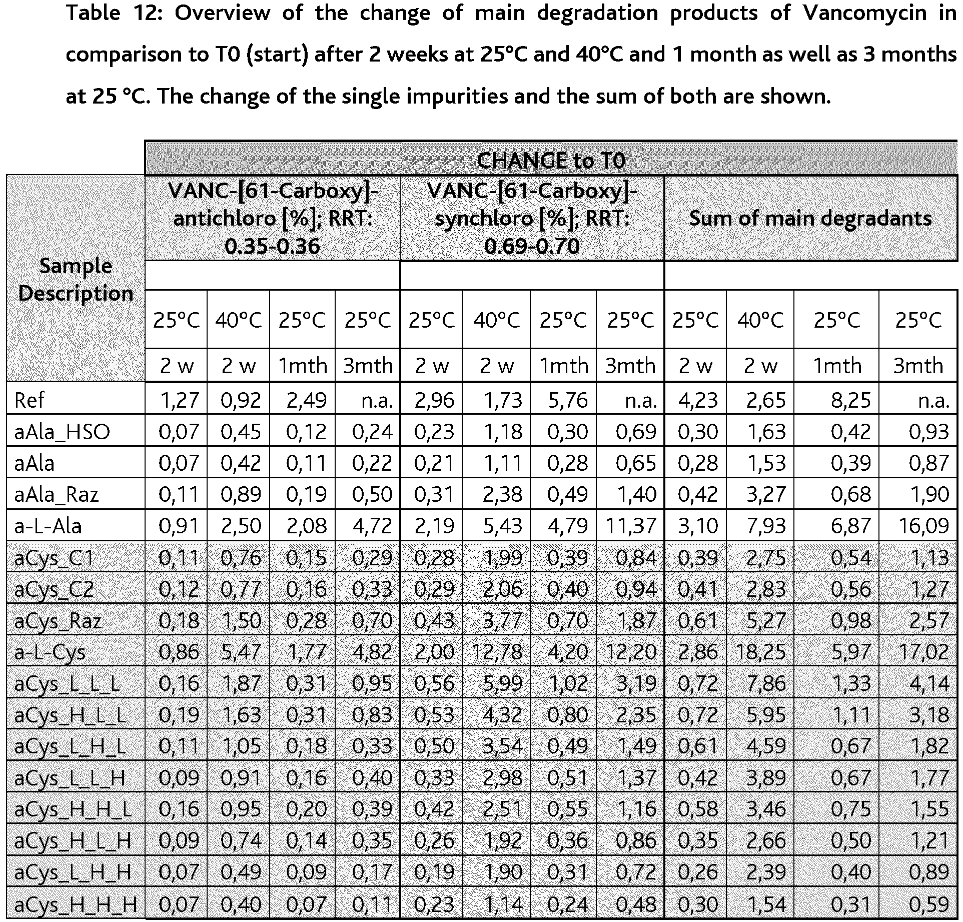

## Claims

1. Aqueous solution comprising glycopeptide antibiotics, selected from the group consisting of vancomycin, telavancin, oritavancin, teicoplanin, dalbavancin and mixtures thereof, especially vancomycin, and N-acetylated D-cysteine (D-aCys), the solution having a pH of from about 4.0 to 7.0.

2. Aqueous solution according to claim 1, **characterised in that** the aqueous solution has a pH of between about 4.0 to about 7.0, especially between about 5.0 and about 6.5, more preferred between about 5.3 and about 6.2, and is present in a suitable container with air as headspace.

3. Aqueous solution according to any of claims 1 or 2 for use as a medicament.

4. Aqueous solution according to any of claims 1 to 3 for use in the treatment of bacterial infections.

5. Method for stabilizing glycopeptide antibiotics, selected from the group consisting of vancomycin, telavancin, oritavancin, teicoplanin, dalbavancin and mixtures thereof, especially vancomycin, which involves addition of N-acetylated D-cysteine (D-aCys) to a solution comprising such glycopeptide antibiotic(s), especially vancomycin, or addition of glycopeptide antibiotic(s), especially vancomycin, to a solution comprising N-acetylated D-cysteine (D-aCys).
